# EUROPEAN PATENT SPECIFICATION

(11) **EP 4 389 088 B1**
(45) Date of publication and mention of the grant of the patent: **28.10.2026**
(21) Application number: 22215788.5
(22) Date of filing: 22.12.2022
(51) Int. Cl.: A61F 13/0203, A61F 13/02

(54) **A MEDICAL DRESSING**
MEDIZINISCHER VERBAND
PANSEMENT MÉDICAL

(43) Date of publication of application: 26.06.2024
(73) Proprietor: Mölnlycke Health Care AB, 431 21 Mölndal (SE)
(72) Inventor: JOHANNISON, Ulf, LANDVETTER (SE); SÖDERSTRÖM, Bengt, MÖLNLYCKE (SE); GERGELY, Ann-Britt, MÖLNDAL (SE)
(74) Representative: Kransell & Wennborg KB

(56) References cited:
- WO-A1-2021/122720
- US-A1- 2005 059 918
- US-A1- 2022 331 167

## Description

### TECHNICAL FIELD

The present disclosure generally relates to a medical dressing comprising an absorbent foam layer having a first side and a second side, wherein the first side of the absorbent foam layer comprises a plurality of compressed depressions. It also relates to a method of manufacturing such a medical dressing.

### BACKGROUND

Wound healing is a complicated process in which destroyed or damaged tissue is replaced with newly produced tissue.

The wound healing process is typically divided into four separate phases; i.e. hemostasis (blood clotting), inflammation, cell proliferation (tissue growth), and tissue remodeling (maturation and cell differentiation). For a wound to heal successfully, all these four phases must be accomplished. The wound healing process is susceptible to failure, which may lead to the formation of non-healing chronic wounds.

To evaluate and secure the progress of wound healing, wound assessment is critical. Clinicians typically assess various wound properties to secure that the wound healing process is proceeding in a proper manner. Factors that are typically assessed by the clinicians include the type of tissue, the level of exudate, as well as the color and characteristics of the exudate.

For example, healthy granulation tissue is pink in color, which is an indication of normal wound healing. Unhealthy granulation is darker red in color, often bleads in contact, and this may be indicative of wound infection. The wound may also be covered with necrotic tissue, which may be identified as a black or dark brown exudate color, and/or slough (dead tissue), which may be yellow in color. Such tissue impairs the wound healing process.

The characteristics of the wound exudate may thus provide clues with respect to the healing potential and progress of the wound, and may assist clinicians to implement appropriate treatment strategies to improve the wound healing process.

Adhesive medical dressings are frequently used in wound care for the purpose of treating wounds. Such medical dressings often comprise an absorbent pad arranged between a backing layer; i.e. a top layer and an adhesive skin contact layer. The absorbent pad often comprises an absorbent foam, e.g. a polyurethane foam.

WO2021/122720A1 relates to an absorbent foam dressing comprising a first and a second coating arranged on the skin-facing side of the absorbent foam.

Absorbent foams, such as polyurethane foams are advantageously utilized for the purpose of absorbing and handling large amounts of wound exudate.

While absorbent foams have a good ability to transport exudate away from the wound site in directions perpendicular to the plane thereof, absorbent foams typically have a relatively poor ability to spread and/or distribute the liquid. This may result in that the wound pad may become locally saturated. As a result, the dressing needs to be replaced, and the wear time of the dressing may be impaired.

Furthermore, absorbent foams, such as polyurethane foams are typically compact in nature, and in order to assess the wound or any characteristics of the wound or wound exudate, the dressing must be removed from the skin.

In view of the above, there is a need for improvements with respect to absorbent foam dressings, particularly with respect to facilitating the wound assessment procedure for clinicians while still securing improved liquid handling properties and an enhanced wear time of the dressing.

### SUMMARY

In view of the above-mentioned problem, it is an object of the present disclosure to provide improvements with respect to improving the wound assessment procedure by the clinicians while simultaneously securing an enhanced wear time of the dressing.

According to a first aspect, there is provided a medical dressing, as defined in claim 1, and comprising an absorbent foam layer having a first side and a second side; the dressing comprising a backing layer facing the first side of the absorbent foam layer and an adhesive skin contact layer facing the second side of the absorbent foam layer, wherein the first side of the absorbent foam layer comprises a plurality of compressed depressions and wherein the backing layer is arranged in direct contact with the first side of the absorbent foam layer.

The plurality of compressed depressions improves the spreading capacity of the absorbent foam layer and secures that the blood and wound liquid exuded by the wound is distributed in an efficient manner within the dressing.

The backing layer is in direct contact with the entire first side of the absorbent foam layer. Accordingly, the backing layer conforms to the contour of the compressed depressions. This way, the surface area of the backing layer, from which exudate can evaporate, is significantly increased. Hence, the wound exudate absorbed by the absorbent foam layer may evaporate through the backing layer in an efficient manner. An improved breathability and moisture vapor permeability is thus achieved.

Furthermore, the direct contact between the first side of the foam layer and the backing layer prevents gaps from forming between the backing layer and the "valleys" defined by the compressed depressions. Such gaps may give rise to liquid reservoirs within the dressing; i.e. instead of being distributed and evaporated from the backing layer, the wound exudate is trapped in the areas of the compressed depressions.

Such a configuration impairs the spreading capacity of the dressing, and may negatively affect the wear time of the dressing. Furthermore, if the exudate remains trapped in the areas; i.e. the "valleys" between the absorbent foam layer and the backing layer, it will become more difficult to visualize the characteristics of the exudate and to identify if (and when) the exudate changes characteristics; i.e. due to wound infection or wound healing regression.

Hence, the compressed depressions in the absorbent foam layer and the directly contacting backing layer improves the visualization of the wound exudate distributed in the wound dressing. The compressed depressions define thinner regions in the absorbent foam, which enables the characteristics of the wound exudate to be analyzed.

The backing layer is heat-laminated onto the first side of the absorbent foam layer.

Accordingly, the backing layer will melt and become directly fused to the first side of the absorbent foam layer. The heat laminated backing layer forms a thin, continuous layer which extends across the entire first side of the absorbent foam layer. The heat-laminated backing layer conforms to the contour of the compressed depressions on the first side of the absorbent foam layer.

The heat-laminated backing layer improves the moisture vapor transmission and breathability of the dressing.

Additionally, the heat laminated has a protective function, and prevents the entry of contaminants into the dressing.

Furthermore, the heat-laminated backing layer yields a visually and tactilely appealing dressing surface. The backing layer will be fused with the absorbent foam layer, yielding a soft and appealing texture.

In exemplary embodiments, the absorbent foam layer comprises a polyurethane foam.

The polyurethane foam is hydrophilic and porous and capable of dealing with large amounts of wound exudate.

In exemplary embodiments, the backing layer comprises polyurethane.

Accordingly, the backing layer and the absorbent foam layer are similar to each other chemically, which improves the heat-lamination process. This way, the direct contact between the backing layer and the first side of the absorbent foam layer is enhanced, and the backing layer conforms to the contour of the plurality of the compressed depressions.

Preferably, the backing layer is a polyurethane film.

In exemplary embodiments, the second side of the absorbent foam layer comprises a plurality of compressed depressions coinciding with the compressed depressions of the first side of the absorbent foam layer.

Accordingly, in the absorbent foam layer areas where the compressed depressions are arranged, the foam is significantly thinner than in the areas free from the compressed depressions.

This arrangement further improves the visibility of the color and characteristics of the wound exudate and improves the wound assessment procedure for clinicians. Instead of removing the entire dressing from the skin of a patient, which would be required if the foam was intact or if the backing layer would not conform to the contour of the compressed depressions, conclusions on the wound healing progress can be drawn while the dressing remains on the patient's skin.

In exemplary embodiments, the compressed depressions on the first and the second side of the absorbent foam layer are spaced apart from one another by non-compressed areas, wherein the thickness, t1, of the absorbent foam layer in the areas forming the compressed depressions, corresponds to 1 to 20 %, preferably from 2 to 10%, more preferably from 3 to 6% of the thickness, t2, of the absorbent foam layer in the non-compressed areas.

This configuration optimizes the balance between efficient exudate distribution and the ability to inspect and assess the wound healing progress.

The absorbent foam layer is typically white or transparent.

Preferably, the absorbent foam layer is white.

The white color of the absorbent foam layer yields an improved contrast with respect to the color of the wound exudate, and secures that the color of the wound exudate can be distinguished in an efficient manner. Accordingly, it will be easy for the clinicians to assess if and when the wound healing process is disrupted. For example, a first volume of exudate may be visualized as pink, but after a while, a second volume of exudate having a darker color or comprising yellow slough may appear, and this is typically an indication that the wound healing process has been impaired.

A transparent foam may, of course, allow for the same type of visual wound inspection. However, a transparent dressing may, by the patient, be experienced as less visually appealing. Accordingly, a slight degree of "masking" the wound, without compromising the ability to draw conclusions if the wound changes character, is generally desired.

In exemplary embodiments, the backing layer has a first side facing the absorbent foam layer and an opposing second side, wherein the second side comprises a plurality of discrete printed elements arranged in a regular pattern across the second side of the backing layer.

The discrete printed elements facilitate the wound assessment for clinicians and caregivers. For example, the clinicians may utilize the discrete printed elements (and the compressed depressions) to note and record the spreading pattern of the wound exudate.

In embodiments where the backing layer is heat-laminated onto the first side of the absorbent layer, the first side of the dressing (i.e. the side facing the clinician) may be used to record and note the wound exudate spreading; i.e. the spreading distance etc. directly on the top surface of the dressing. The printed elements may be utilized to note and record the distance of e.g. a first volume of pink colored exudate, and if present, a second volume of exudate having a different color, and to record the characteristics of the second exudate volume.

The recording of the wound exudate may be utilized to evaluate the progress of the wound healing and identify if additional measures are needed to improve the wound healing or to prevent and/or halt wound infection.

Furthermore, the printed elements and compressed depressions may be used to cut the dressing, if this is desired.

The size of each of the discrete printed elements may be smaller than the size of the compressed depressions on the first side of the absorbent foam layer.

This is beneficial to distinguish the printed elements from the compressed depressions, and to utilize either or both of these features for wound assessment purposes.

For example, the discrete printed elements may be discrete printed dots.

**In** exemplary embodiments, the ratio between the plurality of printed elements and the plurality of compressed depressions on the first side of the absorbent foam layer is from 1:1 to 1:7, preferably from 1:3 to 1:6.

The number of compressed depressions is preferably equal to or higher than the number of printed elements.

A ratio in the above-mentioned range yields a visually appealing impression and facilitates the wound inspection and assessment procedures for the clinicians.

**In** exemplary embodiments, the second side of the backing layer comprises from 3 to 10, preferably from 4 to 7 compressed depressions/cm2.

The amount of compressed depressions in the above mentioned ranges improves the visual wound inspection. Furthermore, the liquid spreading ability of the absorbent foam layer (and the dressing) is improved. In addition, the dressing becomes more compliant and drapeable and conforms to the skin and movement of a patient.

In exemplary embodiments, the adhesive skin contact layer comprises a silicone-based adhesive.

A silicone-based adhesive is gentle to the skin and allows for a non-traumatic removal of the dressing from the skin or the wound of a patient. Furthermore, a silicone-based adhesive secures that the dressing conforms to the skin of a patient.

In exemplary embodiments, the adhesive skin contact layer comprises a first silicone gel coating and a second silicone gel coating; the first silicone gel coating being arranged in contact with the second side of the absorbent foam layer, wherein the first silicone gel coating is a continuous coating and wherein the second silicone gel coating is a discontinuous coating.

The first, continuous silicone gel coating is applied to the second side of the absorbent foam layer and renders this side hydrophobic, thereby preventing wound exudate from flowing back towards the wound site.

The second, discontinuous silicone gel coating improves the adhesion to the skin of the patient, and also allows for wound exudate to enter the absorbent foam layer.

According to another aspect, there is provided a method for manufacturing a medical dressing comprising:
a) providing an absorbent foam layer having a first side and a second side,
b) providing a plurality of compressed depressions on the first side of the absorbent foam layer,
c) heat-laminating a backing layer onto the first side of the absorbent foam layer such that the backing layer is arranged in direct contact with the first side of the absorbent foam layer,
d) providing an adhesive skin contact layer on the second side of the absorbent foam layer.

In exemplary embodiments, the method further comprises the step of:
b') providing a plurality of compressed depressions on the second side of the absorbent foam layer such that the plurality of compressed depressions coincides with the plurality of compressed depressions on the first side of the absorbent foam layer, preferably simultaneously with step b) of providing a plurality of compressed depressions on the first side of the absorbent foam layer.

In exemplary embodiments, the method further comprises the step of:
- stretching the absorbent foam layer comprising the plurality of compressed depressions formed in step b), and optionally in step b') prior to or during step c) of heat-laminating the backing layer onto the first side of the absorbent foam layer.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Figure 1a illustrates a medical dressing according to an exemplary embodiment of the present disclosure.
Figure 1b illustrates a split view of a medical dressing according to an exemplary embodiment, wherein the backing layer is shown separate from the absorbent foam layer.
Figure 2a illustrates a zoomed-in, cross-sectional view of a portion of the dressing in figure 1a.
Figure 2b illustrates a zoomed-in, cross-sectional view of a portion of a dressing not forming part of the present disclosure, wherein the backing layer is not in direct contact with the first side of the absorbent foam layer.

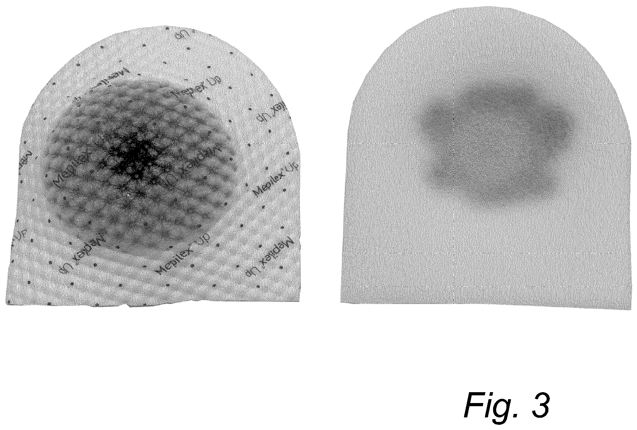

Figure 3 illustrates two are photographs showing the wound exudate distribution as seen from the backing layer of a dressing according to the present disclosure (left photo) compared to a reference dressing (right photo).

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

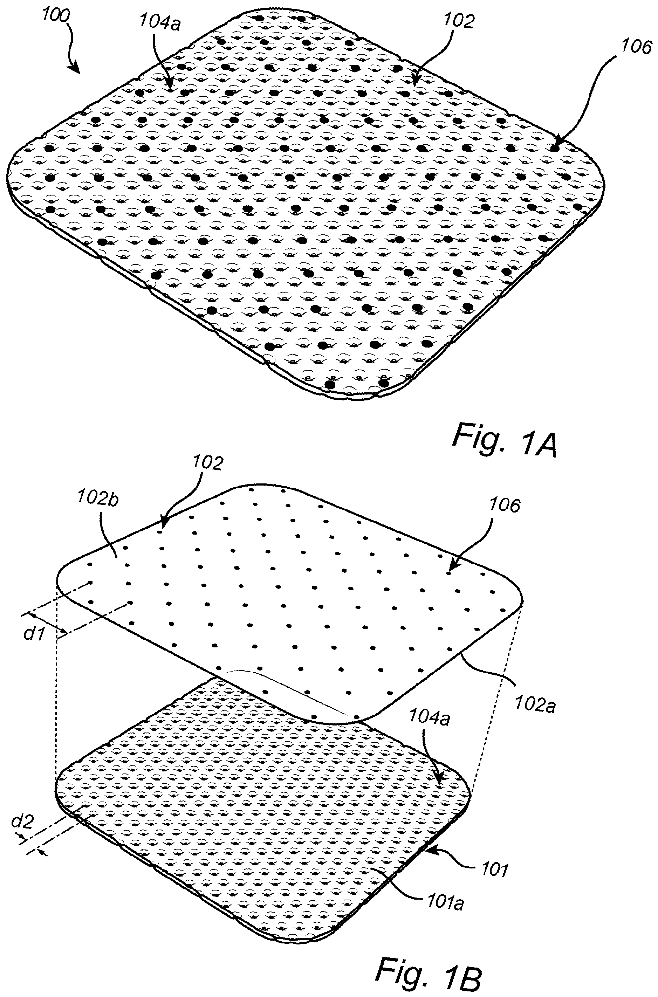

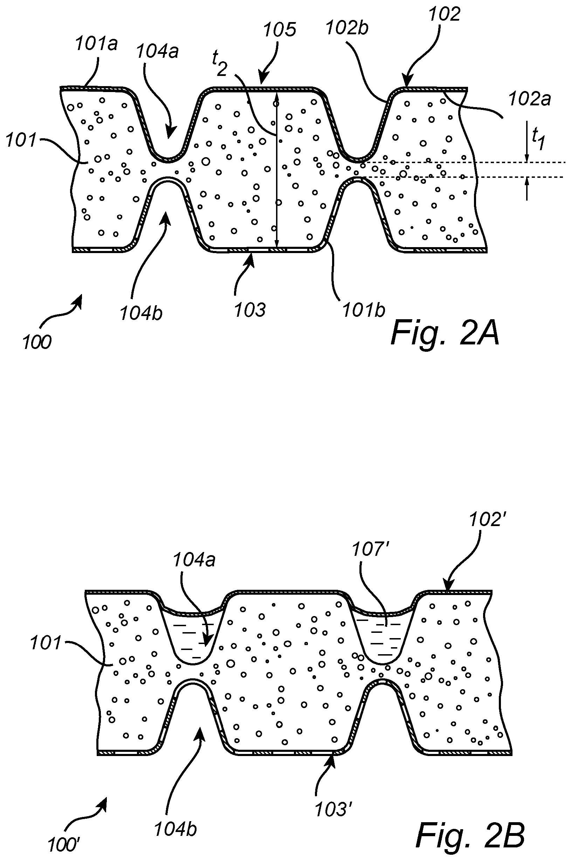

With reference to figures 1A, 1B and 2A, a medical dressing according to an exemplary embodiment of the present disclosure is conceptually illustrated. The medical dressing 100 comprises an absorbent foam layer 101 having a first side 101a and a second side 101b; the dressing 100 comprising a backing layer 102 facing the first side 101a of the absorbent foam layer 101 and an adhesive skin contact layer 103 facing the second side 101b of the absorbent foam layer 101, wherein the first side 101a of the absorbent foam layer 101 comprises a plurality of compressed depressions 104a and wherein the backing layer 102 is arranged in direct contact with the first side 101a of the absorbent foam layer 101.

As used herein, the term "medical dressing" means a dressing used to treat or prevent any type of wound or injury on the skin of a patient. In the context of the present disclosure, wherein wound exudate is to be handled by the dressing, the medical dressing may also be referred to as a wound dressing.

The wound to be treated may be any type of open or closed wound, such as a chronic wound, an acute wound, and post-operative wounds, e.g. a closed incision or scar.

The backing layer 102 is arranged in direct contact with the entire first side 101a of the absorbent foam layer 101 such that the backing layer conforms to the contour of the compressed depressions 104a.

The compressed depressions 104a are preferably arranged in a regular pattern across the first side 101a of the absorbent foam layer 101.

As illustrated in figures 1A and 1B, the plurality of compressed depressions 104a are provided across the entire first side 101a of the absorbent foam layer 101.

A pliable and conformable dressing is thereby provided having an improved liquid spreading capacity. Furthermore, the provision of compressed depressions across the entire first side allows the exudate flow and distribution as well as the characteristics of the exudate (color, texture etc.) to be visualized and monitored by the caregiver or clinician.

As best illustrated in figure 2A, the backing layer 101 is in direct contact with the first side 101a of the absorbent foam layer 101. As can be seen in this figure, the backing layer 101 conforms to the compressed depressions 104a. The backing layer 101 is arranged to follow the contour of the compressed depressions 104a on the first side 101a of the absorbent foam layer 101. No gaps are formed between the backing layer 101 and the first side 101a of the absorbent foam layer 101.

In contrast, and as illustrated in figure 2B, if the backing layer is arranged in a conventional manner, such as by means of adhesives, the backing layer 102' will not remain attached and in direct contact with the first side of the absorbent foam layer 101' in the areas of the compressed depressions 104a'. Accordingly, air gaps 107 will form in these areas, which may turn into liquid reservoirs when wound liquid is exuded from the wound site.

Such liquid reservoirs will impair the spreading and distribution of wound exudate within the dressing. Furthermore, the ability of the wound exudate to evaporate from the backing layer will be impaired. The liquid reservoirs will also impair the visual assessment of the wound exudate and prevent clinicians from monitoring the wound healing progress and draw conclusions with respect to the whether additional treatment measures are required.

In addition, the direct contact between the first side 101a of the absorbent foam layer 101 yields an improved breathability and moisture vapor permeability since the surface area of the backing layer, from which exudate can evaporate, is significantly increased.

The backing layer 102 is heat-laminated onto the first side 101a of the absorbent foam layer 101.

Heat-lamination allows the properties of the absorbent foam layer and the backing layer to be combined. The layers will be fused to each other such that no gaps are formed between the backing layer and the absorbent foam layer. Heat, and optionally pressure, is utilized to bond the two layers together.

If the backing layer is heat-laminated onto the first side of the absorbent foam layer, no adhesive is required. This is beneficial since the presence of adhesives between these layers may impair the breathability of the dressing.

The absorbent foam layer 101 is typically porous and hydrophilic.

The absorbent foam layer 101 may comprise a polyurethane foam, preferably a hydrophilic polyurethane foam. The polyurethane foam is porous and may comprise pores having an average pore size in the range of from 30 and 1000 µm.

An infected wound typically exudes large amounts of exudate, and the wound pad must be capable of properly handling such exudate.

Preferably, the backing layer comprises polyurethane.

For example, the backing layer may be a polyurethane film. The polyurethane film and the polyurethane foam are thus similar to each other chemically, which improves the heat-lamination process.

With the dressing of the present disclosure, a breathable and highly conformable dressing is provided.

As best illustrated in figure 2A, the second side 101b of the absorbent foam layer 101 comprises a plurality of compressed depressions 104b coinciding with the compressed depressions 104a of the first side 101a of the absorbent foam layer 101.

The compressed depressions 104b on the second side 101b are typically coaxial with the compressed depressions 104a on the first side 101a of the absorbent foam layer.

Accordingly, both the first 101a and the second side 101b of the absorbent foam layer 101 have a grooved surface. The surface structure of the first side corresponds to the surface structure of the second side of the absorbent layer (see figure 2A).

The compressed depressions 104a, 104b of the first 101a and the second 101b side of the absorbent foam layer 101 are spaced apart from one another by non-compressed areas 105, wherein the thickness, t1, of the absorbent foam layer 101 in the areas forming the compressed depressions 104a, 104b corresponds 1 to 20 %, preferably from 2 to 10%, more preferably from 3 to 6% of the thickness, t2, of the absorbent foam layer in the non-compressed areas 105.

The thickness, t2, of the absorbent foam layer in the non-compressed areas may be from 2 to 10 mm, e.g from 3 to 6 mm. The thickness is measured in dry conditions.

The thickness, t1, of the absorbent foam layer in the areas forming the compressed depressions may be in the range of from 0.05 to 1.5 mm, e.g. from 0.1 to 0.5 mm. The thickness is measured in dry conditions.

In the regions where the compressed depressions coincide, the wound exudate will be easily visualized and the spreading pattern and exudate characteristics can be monitored.

Typically, the absorbent foam layer is white or transparent.

Preferably, the absorbent foam layer is white.

The contrast between the colored wound exudate and the white foam is thus improved and the color of the wound exudate can be clearly distinguished.

The adhesive skin-contact layer is substantially transparent.

The backing layer may also be transparent. In embodiments where the backing layer is heat-laminated onto the absorbent foam layer, the backing layer will fuse with the foam layer and adopt the texture and appearance of the absorbent foam layer.

As illustrated in figures 1A and 1B, the backing layer 102 has a first side 102a facing the absorbent foam layer 101 and an opposing second side 102b, wherein the second side 102b may comprise a plurality of discrete printed elements 106 arranged in a regular pattern across the second side 102b of the backing layer 102.

The discrete printed elements may be pre-printed on the backing layer prior to heat lamination of the backing layer onto the absorbent foam layer.

The discrete printed elements may be pre-printed on the first or the second side of the backing layer, but can be visualized from the second side of the backing layer.

Typically, the size of each of the discrete printed elements 106 is smaller than the size of the compressed depressions 104a on the first side 101a of the absorbent foam layer.

In figures 1a and 1b, the discrete printed elements 106 are printed dots.

The dots are preferably arranged in regular rows across the second side of the backing layer.

Accordingly, the clinicians can monitor and record the exudate spreading by utilizing the printed dot pattern. For example, the printed dots may be counted, and the spreading pattern may be analyzed and recorded by e.g. making markings directly on the top surface of the dressing, and e.g. drawing lines between the dots defining the wound exudate distribution.

The average distance, d1, between adjacent printed elements on the first side of the absorbent foam layer may be from 0.7 to 1.7 cm, preferably from 0.8 to 1.3 cm.

The average distance between adjacent printed elements, e.g. dots is e preferably about 1 cm. This makes the calculations and ability to record the exudate spreading pattern easier.

The average distance, d2, between adjacent compressed depressions on the first side of the absorbent foam layer is from 0.2 to 0.7 cm, preferably from 0.3 to 0.6 cm.

The ratio between the plurality of printed elements 106 and the plurality of compressed depressions 104a on the first side 101a of the absorbent foam layer 101 may be from 1: 1 to 1:7, preferably from 1:3 to 1:6.

A ratio in the above-mentioned range yields a visually appealing impression and facilitates the wound inspection and assessment procedures for the clinicians.

In exemplary embodiments, the second side of the backing layer comprises from 3 to 10, preferably from 4 to 7 compressed depressions/cm2.

The amount of compressed depressions in the above mentioned ranges improve the visual wound inspection. Furthermore, the liquid spreading ability of the absorbent foam layer (and the dressing) is improved.

In addition, the dressing becomes more compliant and drapeable and conforms to the skin and movement of a patient.

Preferably, the adhesive skin contact layer 103 comprises a silicone-based adhesive.

A silicone-based adhesive is gentle and soft, and allows for a non-traumatic removal of the dressing from the skin or the wound of a patient.

The adhesive skin contact layer 103 is typically coated onto the second side 101b of the absorbent foam layer. The adhesive skin contact layer is in direct contact with the second side of the absorbent foam layer. Accordingly, no gaps which may give rise to liquid reservoirs are formed in the dressing.

The adhesive skin contact layer 102 may comprise a first silicone gel coating and a second silicone gel coating; the first silicone gel coating being arranged in contact with the second side 101b of the absorbent foam layer 101, wherein the first silicone gel coating is a continuous coating and wherein the second silicone gel coating is a discontinuous coating.

The first silicone gel coating is a continuous coating, and is applied directly to the second side of the absorbent foam layer 101b. The first silicone gel coating may, apart from providing adhesive contact to the skin and/or wound, also render the hydrophilic absorbent foam layer hydrophobic. This is beneficial to prevent wound exudate from flowing back towards the wound site.

The second silicone gel coating is discontinuous and is applied onto the first silicone gel coating. The second silicone gel coating improves the adhesion to the skin of the patient, and also facilitates entry of wound exudate into the absorbent foam layer.

According to another aspect, there is provided a method for manufacturing a medical dressing 100 comprising:
a) providing an absorbent foam layer 101 having a first side 101a and a second side 101b,
b) providing a plurality of compressed depressions 104a on the first side 101a of the absorbent foam layer 101,
c) heat-laminating a backing layer 102 onto the first side 101a of the absorbent foam layer 101 such that the backing layer 102 is arranged in direct contact with the first side 101 of the absorbent foam layer 101,
d) providing an adhesive skin contact layer 103 on the second side 101b of the absorbent foam layer 101.

Preferably, the absorbent foam layer 101 comprises a polyurethane foam. The polyurethane foam may be provided by means known to the skilled person.

The provision of the plurality of compressed depressions 104a on the first side 101a of the absorbent foam layer is not limited to a specific technique, but any technique may be utilized.

Preferably, the method further comprises the step of:
b') providing a plurality of compressed depressions 104b on the second side 101b of the absorbent foam layer 101 such that the plurality of compressed depressions 104b coincide with the plurality of compressed depressions 104a on the first side 101a of the absorbent foam layer 101, preferably simultaneously with step b) of providing a plurality of compressed depressions 104a on the first side 101a of the absorbent foam layer 101.

The compressed depressions on the first and second side of the absorbent foam layer may be provided by feeding the absorbent foam layer between a counter roller having a pattern of protrusions projecting from its outer surface and a horn of an ultrasonic welding device.

The backing layer 102 may be applied onto the first side 101a of the absorbent foam layer 101 by heat-lamination, such that the backing layer 102 is arranged in direct contact with the first side 101 of the absorbent foam layer 101.

The backing layer 102 is typically thin and may be applied to the absorbent foam layer 101 by the aid of a roller. The backing layer may then be heated to a temperature which allows the backing layer to melt and to form a continuous layer on top of the absorbent foam layer, which conforms to the contour of the first side of the absorbent foam layer. Preferably, the method further comprises the step of:
- stretching the absorbent foam layer 101 comprising the plurality of compressed depressions formed in step b), and optionally in step b') prior to or during the step c) of heat-laminating the backing layer 102 onto the first side 101a of the absorbent foam layer 101.

The absorbent foam layer 101 may be stretched from 5% to 30%, e.g. from 15 to 25% in the lateral (x) and longitudinal (y) directions.

An excess amount of backing layer material is thus provided when the absorbent foam layer subsequently retracts. Pin-holes in the backing layer are thereby prevented. Accordingly, a continuous and protective backing layer which conforms to the first side of the absorbent foam layer is accomplished.

The adhesive skin contact layer typically comprises a silicone-based adhesive, and may be coated, e.g. by means of transfer coating onto the second side of the absorbent foam layer.

### Example 1: Preparation of a medical dressing

An absorbent foam layer according to the present disclosure was prepared from a web of uncompressed polyurethane foam. The uncompressed PU foam had a thickness of about 5 mm. To form the plurality of compressed depressions, the uncompressed PU foam was fed between a counter roller having a pattern of protrusions projecting from its outer surface and an ultrasonic horn.

The horn was moved towards and away from the counter roller at an ultrasonic frequency. Accordingly, heat was developed in the material due to internal friction. The heat was thus concentrated to the foam disposed between the tips of the protrusions of the counter roller and the horn. The energy delivered by the ultrasonic welding device was so high that the material between the tips of the protrusions and the horn was fused together so that no cells or very small cells were left in these portions of the web after the compression step with the ultrasonic welding device.

Subsequently, a polyurethane film was heat laminated onto the first side of the absorbent foam layer. The polyurethane film was applied, and the dressing substrates were fed between two rolls, wherein the roll facing the backing layer was heated to a temperature of 140 degrees. The backing layer and the absorbent layer were pressed against each other. Accordingly, the polyurethane film was melted and formed a continuous backing layer conforming to the shape of the compressed depressions on the first side of the absorbent foam pad layer. The absorbent foam layer was in a stretched state during heat-lamination.

Subsequently, an adhesive silicone gel layer was applied to the second side of the absorbent foam layer by means of transfer coating.

### Example 2: Visualization of wound exudate

In order to evaluate the dressing's effectiveness in facilitating wound inspection, simulated comparative tests were carried out.

The dressing prepared according to Example 1 (hereinafter referred to as Dressing A) was compared with a foam dressing with similar constituents. The comparative dressing (referred to as Dressing B) differed from dressing A in that the polyurethane foam was not compressed. Instead, the absorbent foam layer utilized was a 5 mm thick, uncompressed polyurethane foam.

The dressing samples were placed on a flat support. The flat support contained a central hole. The dressing samples were placed such that they were overlying the hole of the support. A tube was attached to the central portion of the dressing sample (through the hole). The tube was connected to a syringe pump, and 2.5 ml red ink (representing a first volume of exudate) was pumped into the dressing at a flow rate of 0.5 ml/minutes. After 50 seconds, a blue ink (representing a second volume of exudate) was pumped into the dressing at a flow rate of 0.5 ml/minutes. The total volume of blue ink was 1 ml.

With the dressing of the present disclosure (dressing A), the different colored liquids could easily be distinguished, and the degree of distribution could be evaluated (see left photo). For dressing B, the different colored liquids were difficult to distinguish, and the visual inspection of the exudate color and distribution was significantly impaired (see right photo).

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A medical dressing (100) comprising an absorbent foam layer (101) having a first side (101a) and a second side (101b); said dressing (100) comprising a backing layer (102) facing said first side (101a) of said absorbent foam layer (101) and an adhesive skin contact layer (103) facing said second side (101b) of said absorbent foam layer (101), wherein said first side (101a) of said absorbent foam layer (101) comprises a plurality of compressed depressions (104a), **characterized in that** said backing layer (102) is arranged in direct contact with said first side (101a) of said absorbent foam layer (101), wherein said backing layer (102) is heat-laminated onto said first side (101a) of said absorbent foam layer (101).

2. The medical dressing (100) according to claim 1, wherein said absorbent foam layer (101) comprises a polyurethane foam.

3. The medical dressing according to claim 1 or claim 2, wherein said backing layer (102) comprises polyurethane.

4. The medical dressing (100) according to any one of the preceding claims, wherein said second side (101b) of said absorbent foam layer (101) comprises a plurality of compressed depressions (104b) coinciding with said compressed depressions (104a) of said first side (101a) of said absorbent foam layer (101).

5. The medical dressing (100) according to claim 4, wherein said compressed depressions (104a, 104b) of said first (101a) and said second (101b) side of said absorbent foam layer (101) are spaced apart from one another by non-compressed areas (105), wherein the thickness, t1, of said absorbent foam layer (101) in the areas forming said compressed depressions (104a, 104b) corresponds to 1 to 20 %, preferably from 2 to 10%, more preferably from 3 to 6% of the thickness, t2, of said absorbent foam layer in said non-compressed areas (105).

6. The medical dressing (100) according to any one of the preceding claims, wherein said absorbent foam layer is white or transparent.

7. The medical dressing (100) according to any one of the preceding claims, wherein said backing layer (102) has a first side (102a) facing said absorbent foam layer (101) and an opposing second side (102b), wherein said second side (102b) comprises a plurality of discrete printed elements (106) arranged in a regular pattern across said second side (102b) of said backing layer (102).

8. The medical dressing (100) according to any one of the preceding claims, wherein the size of each of said discrete printed elements (106) is smaller than the size of said compressed depressions (104a) on said first side (101a) of said absorbent foam layer.

9. The medical dressing (100) according to claim 7 or claim 8, wherein the ratio between said plurality of printed elements (106) and said plurality of compressed depressions (104a) on said first side (101a) of said absorbent foam layer (101) is from 1:1 to 1:7, preferably from 1:3 to 1:6.

10. The medical dressing (100) according to any one of the preceding claims, wherein said second side (102b) of said backing layer (102) comprises from 3 to 10, preferably from 4 to 7 compressed depressions/cm2.

11. The medical dressing (100) according to any one of the preceding claims, wherein said adhesive skin contact layer (103) comprises a silicone-based adhesive.

12. The medical dressing (100) according to any one of the preceding claims, wherein said adhesive skin contact layer (103) comprises a first silicone gel coating and a second silicone gel coating; said first silicone gel coating being arranged in contact with said second side (101b) of said absorbent foam layer (101), wherein said first silicone gel coating is a continuous coating and wherein second silicone gel coating is a discontinuous coating.

13. A method for manufacturing a medical dressing (100) comprising:
a) providing an absorbent foam layer (101) having a first side (101a) and a second side (101b),
b) providing a plurality of compressed depressions (104a) on said first side (101a) of said absorbent foam layer (101),
c) heat-laminating a backing layer (102) onto said first side (101a) of said absorbent foam layer (101) such that said backing layer (102) is arranged in direct contact with said first side (101) of said absorbent foam layer (101),
d) providing an adhesive skin contact layer (103) on said second side (101b) of said absorbent foam layer (101).

14. The method according to claim 13, wherein said method further comprises the step of:
b') providing a plurality of compressed depressions (104b) on said second side (101b) of said absorbent foam layer (101) such that said plurality of compressed depressions (104b) coincide with the plurality of compressed depressions (104a) on said first side (101a) of said absorbent foam layer (101), preferably simultaneously with said step b) of providing said plurality of compressed depressions (104a) on said first side (101a) of said absorbent foam layer (101).

15. The method according to claim 13 or claim 14, wherein said method further comprises the step of:
- stretching the absorbent foam layer (101) comprising said plurality of compressed depressions formed in said step b), and optionally in said step b') prior to or during said step c) of heat-laminating said backing layer (102) onto said first side (101a) of said absorbent foam layer (101).

## Patentansprüche

1. Medizinischer Verband (100), der eine absorbierende Schaumschicht (101) mit einer ersten Seite (101a) und einer zweiten Seite (101b) umfasst; wobei der Verband (100) eine Trägerschicht (102), die der ersten Seite (101a) der absorbierenden Schaumschicht (101) zugewandt ist, und eine haftende Hautkontaktschicht (103) umfasst, die der zweiten Seite (101b) der absorbierenden Schaumschicht (101) zugewandt ist, wobei die erste Seite (101a) der absorbierenden Schaumschicht (101) eine Vielzahl von eingepressten Vertiefungen (104a) umfasst, **dadurch gekennzeichnet, dass** die Trägerschicht (102) in direktem Kontakt mit der ersten Seite (101a) der absorbierenden Schaumschicht (101) angeordnet ist, wobei die Trägerschicht (102) unter Wärmeeinwirkung auf die erste Seite (101a) der absorbierenden Schaumschicht (101) aufkaschiert ist.

2. Medizinischer Verband (100) nach Anspruch 1, wobei die absorbierende Schaumschicht (101) einen Polyurethanschaum umfasst.

3. Medizinischer Verband nach Anspruch 1 oder Anspruch 2, wobei die Trägerschicht (102) Polyurethan umfasst.

4. Medizinischer Verband (100) nach einem der vorhergehenden Ansprüche, wobei die zweite Seite (101b) der absorbierenden Schaumschicht (101) eine Vielzahl von eingepressten Vertiefungen (104b) umfasst, die mit den eingepressten Vertiefungen (104a) der ersten Seite (101a) der absorbierenden Schaumschicht (101) übereinstimmen.

5. Medizinischer Verband (100) nach Anspruch 4, wobei die eingepressten Vertiefungen (104a, 104b) der ersten (101a) und der zweiten (101b) Seite der absorbierenden Schaumschicht (101) durch nicht eingepresste Bereiche (105) voneinander beabstandet sind, wobei die Dicke, t1, der absorbierenden Schaumschicht (101) in den Bereichen, die die eingepressten Vertiefungen (104a, 104b) bilden, 1 bis 20 %, vorzugsweise 2 bis 10 %, bevorzugter 3 bis 6 % der Dicke, t2, der absorbierenden Schaumschicht in den nicht eingepressten Bereichen (105) entspricht.

6. Medizinischer Verband (100) nach einem der vorhergehenden Ansprüche, wobei die absorbierende Schaumschicht weiß oder durchsichtig ist.

7. Medizinischer Verband (100) nach einem der vorhergehenden Ansprüche, wobei die Trägerschicht (102) eine erste Seite (102a), die der absorbierenden Schaumschicht (101) zugewandt ist, und eine gegenüberliegende zweite Seite (102b) aufweist, wobei die zweite Seite (102b) eine Vielzahl von einzelnen aufgedruckten Elementen (106) umfasst, die in einem regelmäßigen Muster über die zweite Seite (102b) der Trägerschicht (102) hinweg angeordnet ist.

8. Medizinischer Verband (100) nach einem der vorhergehenden Ansprüche, wobei jedes der einzelnen aufgedruckten Elemente (106) kleiner ist als die eingepressten Vertiefungen (104a) auf der ersten Seite (101a) der absorbierenden Schaumschicht.

9. Medizinischer Verband (100) nach Anspruch 7 oder Anspruch 8, wobei das Verhältnis zwischen der Vielzahl von aufgedruckten Elementen (106) und der Vielzahl von eingepressten Vertiefungen (104a) auf der ersten Seite (101a) der absorbierenden Schaumschicht (101) von 1:1 bis 1:7, vorzugsweise von 1:3 bis 1:6 beträgt.

10. Medizinischer Verband (100) nach einem der vorhergehenden Ansprüche, wobei die zweite Seite (102b) der Trägerschicht (102) von 3 bis 10, vorzugsweise von 4 bis 7 eingepresste Vertiefungen/cm² umfasst.

11. Medizinischer Verband (100) nach einem der vorhergehenden Ansprüche, wobei die haftende Hautkontaktschicht (103) einen Klebstoff auf Silikonbasis umfasst.

12. Medizinischer Verband (100) nach einem der vorhergehenden Ansprüche, wobei die haftende Hautkontaktschicht (103) eine erste Silikongelbeschichtung und eine zweite Silikongelbeschichtung umfasst; wobei die erste Silikongelbeschichtung im Kontakt mit der zweiten Seite (101b) der absorbierenden Schaumschicht (101) angeordnet ist, wobei die erste Silikongelbeschichtung eine durchgängige Beschichtung ist und wobei die zweite Silikongelbeschichtung eine unterbrochene Beschichtung ist.

13. Verfahren zum Herstellen eines medizinischen Verbands (100), umfassend:
a) Bereitstellen einer absorbierenden Schaumschicht (101), die eine erste Seite (101a) und eine zweite Seite (101b) aufweist;
b) Bereitstellen einer Vielzahl von eingepressten Vertiefungen (104a) auf der ersten Seite (101a) der absorbierenden Schaumschicht (101),
c) unter Wärmeeinwirkung Aufkaschieren einer Trägerschicht (102) auf die erste Seite (101a) der absorbierenden Schaumschicht (101), sodass die Trägerschicht (102) im direkten Kontakt mit der ersten Seite (101) der absorbierenden Schaumschicht (101) angeordnet ist,
d) Bereitstellen einer haftenden Hautkontaktschicht (103) auf der zweiten Seite (101b) der absorbierenden Schaumschicht (101).

14. Verfahren nach Anspruch 13, wobei das Verfahren ferner folgenden Schritt umfasst:
b') Bereitstellen einer Vielzahl von eingepressten Vertiefungen (104b) auf der zweiten Seite (101b) der absorbierenden Schaumschicht (101), sodass die Vielzahl von eingepressten Vertiefungen (104b) mit der Vielzahl von eingepressten Vertiefungen (104a) auf der ersten Seite (101a) der absorbierenden Schaumschicht (101) übereinstimmt, vorzugsweise gleichzeitig mit Schritt b) zum Bereitstellen der Vielzahl von eingepressten Vertiefungen (104a) auf der ersten Seite (101a) der absorbierenden Schaumschicht (101).

15. Verfahren nach Anspruch 13 oder Anspruch 14, wobei das Verfahren ferner folgenden Schritt umfasst:
- Dehnen der absorbierenden Schaumschicht (101), die die Vielzahl von eingepressten Vertiefungen umfasst, die in Schritt b), und optional in Schritt b') gebildet wurden, vor oder während Schritt c) zum Aufkaschieren der Trägerschicht (102) auf die erste Seite (101a) der absorbierenden Schaumschicht (101) unter Wärmeeinwirkung.

## Revendications

1. Pansement médical (100) comprenant une couche de mousse absorbante (101) présentant une première face (101a) et une seconde face (101b) ; ledit pansement (100) comprenant une couche support (102) faisant face à ladite première face (101a) de ladite couche de mousse absorbante (101) et une couche adhésive de contact avec la peau (103) faisant face à ladite seconde face (101b) de ladite couche de mousse absorbante (101), ladite première face (101a) de ladite couche de mousse absorbante (101) comprenant une pluralité de dépressions comprimées (104a),
**caractérisé en ce que** ladite couche support (102) est disposée en contact direct avec ladite première face (101a) de ladite couche de mousse absorbante (101), ladite couche support (102) étant stratifiée thermiquement sur ladite première face (101a) de ladite couche de mousse absorbante (101).

2. Pansement médical (100) selon la revendication 1, dans lequel ladite couche de mousse absorbante (101) comprend une mousse de polyuréthane.

3. Pansement médical selon la revendication 1 ou la revendication 2, dans lequel ladite couche support (102) comprend du polyuréthane.

4. Pansement médical (100) selon l'une quelconque des revendications précédentes, dans lequel ladite seconde face (101b) de ladite couche de mousse absorbante (101) comprend une pluralité de dépressions comprimées (104b) coïncidant avec lesdites dépressions comprimées (104a) de ladite première face (101a) de ladite couche de mousse absorbante (101).

5. Pansement médical (100) selon la revendication 4, dans lequel lesdites dépressions comprimées (104a, 104b) de ladite première face (101a) et de ladite seconde face (101b) de ladite couche de mousse absorbante (101) sont espacées l'une de l'autre par des zones non comprimées (105), l'épaisseur t1 de ladite couche de mousse absorbante (101) dans les zones formant lesdites dépressions comprimées (104a, 104b) équivalant à 1 à 20 %, de préférence 2 à 10 %, plus préférablement 3 à 6 % de l'épaisseur t2, de ladite couche de mousse absorbante dans lesdites zones non comprimées (105).

6. Pansement médical (100) selon l'une quelconque des revendications précédentes, dans lequel ladite couche de mousse absorbante est blanche ou transparente.

7. Pansement médical (100) selon l'une quelconque des revendications précédentes, dans lequel ladite couche support (102) a une première face (102a) faisant face à ladite couche de mousse absorbante (101) et une seconde face opposée (102b), ladite seconde face (102b) comprenant une pluralité d'éléments imprimés discrets (106) agencés selon un motif régulier en travers de ladite seconde face (102b) de ladite couche support (102).

8. Pansement médical (100) selon l'une quelconque des revendications précédentes, dans lequel la taille de chacun desdits éléments imprimés discrets (106) est inférieure à la taille desdites dépressions comprimées (104a) sur ladite première face (101a) de ladite couche de mousse absorbante.

9. Pansement médical (100) selon la revendication 7 ou la revendication 8, dans lequel le rapport entre ladite pluralité d'éléments imprimés (106) et ladite pluralité de dépressions comprimées (104a) sur ladite première face (101a) de ladite couche de mousse absorbante (101) est de 1 :1 à 1 :7, de préférence de 1 :3 à 1 :6.

10. Pansement médical (100) selon l'une quelconque des revendications précédentes, dans lequel ladite seconde face (102b) de ladite couche support (102) comprend 3 à 10, de préférence 4 à 7 dépressions comprimées/cm².

11. Pansement médical (100) selon l'une quelconque des revendications précédentes, dans lequel ladite couche adhésive de contact avec la peau (103) comprend un adhésif à base de silicone.

12. Pansement médical (100) selon l'une quelconque des revendications précédentes, dans lequel ladite couche adhésive de contact avec la peau (103) comprend un premier revêtement de gel de silicone et un second revêtement de gel de silicone ; ledit premier revêtement de gel de silicone étant agencé en contact avec ladite seconde face (101b) de ladite couche de mousse absorbante (101), ledit premier revêtement de gel de silicone étant un revêtement continu et le second revêtement de gel de silicone étant un revêtement discontinu.

13. Procédé de fabrication d'un pansement médical (100) consistant à :
a) prévoir une couche de mousse absorbante (101) ayant une première face (101a) et une seconde face (101b),
b) prévoir une pluralité de dépressions comprimées (104a) sur ladite première face (101a) de ladite couche de mousse absorbante (101),
c) laminer à chaud une couche support (102) sur ladite première face (101a) de ladite couche de mousse absorbante (101) de sorte que ladite couche support (102) soit disposée en contact direct avec ladite première face (101) de ladite couche de mousse absorbante (101),
d) prévoir une couche adhésive de contact avec la peau (103) sur ladite seconde face (101b) de ladite couche de mousse absorbante (101).

14. Procédé selon la revendication 13, dans lequel ledit procédé comprend en outre l'étape consistant à :
b') prévoir une pluralité de dépressions comprimées (104b) sur ladite seconde face (101b) de ladite couche de mousse absorbante (101) de sorte que ladite pluralité de dépressions comprimées (104b) coïncident avec la pluralité de dépressions comprimées (104a) sur ladite première face (101a) de ladite couche de mousse absorbante (101), de préférence simultanément à ladite étape b) de prévision de ladite pluralité de dépressions comprimées (104a) sur ladite première face (101a) de ladite couche de ladite couche de mousse absorbante (101).

15. Procédé selon la revendication 13 ou la revendication 14, dans lequel ledit procédé comprend en outre l'étape consistant à :
- étirer la couche de mousse absorbante (101) comprenant ladite pluralité de dépressions comprimées formées dans ladite étape b), et éventuellement dans ladite étape b') avant ou pendant ladite étape c) de stratification thermique de ladite couche support (102) sur ladite première face (101a) de ladite couche de mousse absorbante (101).
